(19) Europäisches Patentamt European Patent Office Office européen des brevets

(11) **EP 4 737 586 A1**

(12) **EUROPEAN PATENT APPLICATION**

(43) Date of publication:
06.05.2026 Bulletin 2026/19

(21) Application number: 25212998.6

(22) Date of filing: 03.11.2025

(51) International Patent Classification (IPC):
*C12Q 1/26* (2006.01)    *C12Q 1/28* (2006.01)
*G01N 33/542* (2006.01)    *G01N 33/58* (2006.01)

(52) Cooperative Patent Classification (CPC):
**C12Q 1/28; C12Q 1/26; G01N 33/542;
G01N 33/582**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **05.11.2024 JP 2024193972**

(71) Applicant: **Canon Kabushiki Kaisha
Tokyo 146-8501 (JP)**

(72) Inventors:
• **YAMAUCHI, Fumio
Tokyo (JP)**
• **KAKEGAWA, Norishige
Tokyo (JP)**
• **KITAGAWA, Kenji
Tokyo (JP)**
• **NOMOTO, Tsuyoshi
Tokyo (JP)**

(74) Representative: **WESER & Kollegen
Patentanwälte PartmbB
Radeckestraße 43
81245 München (DE)**

(54) **METHOD OF DETECTING TARGET SUBSTANCE AND REAGENT FOR INSPECTION OF TARGET SUBSTANCE**

(57)    Provided are a highly sensitive detection method for a target substance and a reagent for inspection thereof. Specifically, provided is a method of detecting a target substance that is an enzyme reaction-related substance, the method including: a first step of mixing a specimen and a reagent to obtain a liquid sample containing hydrogen peroxide, a peroxidase (40), a phenol derivative, a luminescent particle (10) and hydrophilic polymers (30) each having a binding functional group; a second step of generating an aggregate (50) of the hydrophilic polymers (30) including the luminescent particle (10) through the binding functional groups of the hydrophilic polymers (30) based on a reaction of the hydrogen peroxide, the peroxidase (40) and the phenol derivative, which occurs when the target substance is present in the liquid sample; and a third step of obtaining a value related to fluorescence anisotropy of the liquid sample in the second step.

FIG. 1

| FIRST STEP OF MIXING SPECIMEN THAT MAY CONTAIN TARGET SUBSTANCE AND REAGENT TO OBTAIN LIQUID SAMPLE CONTAINING HYDROGEN PEROXIDE, PEROXIDASE, PHENOL DERIVATIVE, LUMINESCENT PARTICLES AND PLURALITY OF HYDROPHILIC POLYMERS EACH HAVING BINDING FUNCTIONAL GROUP | S1001 |

| SECOND STEP OF GENERATING AGGREGATE OF HYDROPHILIC POLYMERS INCLUDING LUMINESCENT PARTICLES THROUGH BINDING OF BINDING FUNCTIONAL GROUPS OF HYDROPHILIC POLYMERS BASED ON REACTION OF HYDROGEN PEROXIDE, PEROXIDASE AND PHENOL DERIVATIVE, WHICH OCCURS WHEN TARGET SUBSTANCE IS PRESENT IN LIQUID SAMPLE | S1002 |

| THIRD STEP OF OBTAINING VALUE RELATED TO FLUORESCENCE ANISOTROPY OF LIQUID SAMPLE IN STEP OF GENERATING AGGREGATE OF HYDROPHILIC POLYMERS CONTAINING LUMINESCENT PARTICLE | S1003 |

| FOURTH STEP OF DETECTING TARGET SUBSTANCE BASED ON VALUE RELATED TO FLUORESCENCE ANISOTROPY | S1004 |

## Description

TECHNICAL FIELD

The present disclosure relates to a method of detecting a target substance and a reagent for inspection of a target substance.

BACKGROUND

Enzymes have a role of catalyzing chemical reactions in a living body, and measurement of enzyme activity is extremely important in the fields of not only medical research of enzymes but also inspection of enzymes, clinical inspections utilizing enzymes as reagents and substance production utilizing enzymes.

Hitherto, enzyme activity has been measured by various methods. In general, a method using a natural or synthetic substrate for an individual enzyme is performed. In the method using a synthetic substrate, the enzyme activity can be measured by utilizing the enzyme activity and optically detecting and measuring a dye that is liberated from the synthetic substrate by the action of the enzyme. Alternatively, for example, a substrate is used in which luminescence is weak before the action of the enzyme, but the luminescence intensity increases after the action.

However, when the enzyme concentration is extremely low, the amount of a fluorescent dye liberated from the synthetic substrate is also small, and hence the luminescence from the fluorescent dye weakens. Thus, there has been a problem in that the measurement of the enzyme activity becomes difficult, or measurement accuracy decreases. In addition, the fluorescent dye is liable to be affected by the surrounding environment, and for example, in a liquid sample containing a large amount of contaminants such as blood, the dye adsorbs to contaminants, such as proteins and lipids, and the fluorescent properties of the substrate change. As a result, high-sensitivity measurement becomes difficult in some cases. Further, in order to eliminate the influence of contaminants, it is possible to separate the enzyme that is a target substance or the contaminants, but the separation work is complicated, and the measurement time may lengthen.

Fluorescence polarization measurement is known as one of the methods of measuring enzyme activity. The fluorescence polarization measurement, which utilizes fluorescence polarization, is a method of measuring the rotational motion of a fluorescent substance. When the fluorescent substance is excited, the polarization of its fluorescence (also referred to as "degree of fluorescence polarization") changes in accordance with the rotational motion of the fluorescent substance. That is, the method utilizes the fact that when the fluorescent substance does not rotate, polarized luminescence is observed, whereas when the fluorescent substance freely rotates, fluorescence is radiated in all planes, and the polarization is eliminated. A feature of the fluorescence polarization measurement is that in the assay, the degree of fluorescence polarization is used as an indicator, not the luminescence intensity of the fluorescence. Another feature is that the method does not require a separation or washing operation. That is, a homogeneous assay is possible, and a specimen can be added to a solution and measured as it is. Thus, complicated separation work is unnecessary, and measurement can be performed in a short period of time.

Methods of measuring enzyme activity by the fluorescence polarization measurement have heretofore been disclosed. In Japanese Patent Laid-Open No. H10-099097, there is a disclosure of the measurement of the activity of a protease, which is a protein-degrading enzyme. The measurement utilizes the fact that when a substrate molecule having a luminescent substance is cleaved by the protease, the size of the luminescent substance decreases, and the mobility of the luminescent substance changes. In U.S. Patent Application Publication No. 2008/0145880, there is a disclosure of the measurement of the activity of an enzyme that catalyzes a phosphoric acid modification, including a kinase, a phosphatase, a cyclase and a phosphodiesterase.

In U.S. Patent Application Publication No. 2017/0239356, there is a disclosure of a technology of producing a hydrogel with horseradish peroxidase (HRP), which is an oxidoreductase. U.S. Patent Application Publication No. 2017/0239356 is directed to encapsulate living cells in a hydrogel under mild conditions using HRP for medical applications. However, it has been difficult to measure a substance related to an enzyme reaction of a peroxidase in a simple manner and with high sensitivity.

SUMMARY

The present disclosure in its first aspect provides a method of detecting a target substance that is a substance related to an enzyme reaction, the method including: a first step of mixing a specimen that may contain the target substance and a reagent to obtain a liquid sample containing hydrogen peroxide, a peroxidase, a phenol derivative, a luminescent particle and a plurality of hydrophilic polymers each having a binding functional group; a second step of generating an aggregate of the hydrophilic polymers including the luminescent particle through binding of the binding functional groups of the hydrophilic polymers based on a reaction of the hydrogen peroxide, the peroxidase and the phenol derivative, which occurs when the target substance is present in the liquid sample; and a third step of obtaining a value related to fluorescence anisotropy of the liquid sample in the step of generating the aggregate of the hydrophilic polymers containing the luminescent particle as specified in claim 1. Optional features are specified in claims 2 to 11.

Further, the present disclosure in its second aspect provides a reagent for inspection of a target substance using measurement of a value related to fluorescence anisotropy, wherein the target substance is a peroxidase, and the reagent contains hydrogen peroxide, a phenol derivative, a luminescent particle and a hydrophilic polymer having a binding functional group; wherein the target substance is hydrogen peroxide, and the reagent contains a peroxidase, a phenol derivative, a luminescent particle and a hydrophilic polymer having a binding functional group; or wherein the target substance is a phenol derivative, and the reagent contains a peroxidase, hydrogen peroxide, a luminescent particle and a hydrophilic polymer having a binding functional group as specified in claim 12. Optional features are specified in claim 13.

Further, the present disclosure in its third aspect provides a reagent for inspection of a target substance using measurement of a value related to fluorescence anisotropy, wherein the target substance is an oxidase, and the reagent contains a substrate for the oxidase, a peroxidase, hydrogen peroxide, a phenol derivative, a luminescent particle and a hydrophilic polymer having a binding functional group; or wherein the target substance is a substrate for an oxidase, and the reagent contains the oxidase, a peroxidase, hydrogen peroxide, a phenol derivative, a luminescent particle and a hydrophilic polymer having a binding functional group as specified in claim 14.

According to the present disclosure, a substance related to a peroxidase enzyme reaction can be measured in a simple manner and with high sensitivity. In particular, the enzyme activity can be measured with high sensitivity and rapidly even when the amount of an enzyme is small or the enzyme activity is low.

Features of the present disclosure will become apparent from the following description of embodiments with reference to the attached drawings. The following description of embodiments is described by way of example.

BRIEF DESCRIPTION OF THE DRAWINGS

Fig. 1 is a diagram for illustrating the steps of a method of detecting a target substance according to one aspect of the present disclosure.
Fig. 2 is a diagram for illustrating a highly sensitive measurement method for a peroxidase using the formation of an aggregate of hydrophilic polymers according to an embodiment of the present disclosure.
Fig. 3 is a diagram for illustrating a measurement method for glucose oxidase using the formation of an aggregate of hydrophilic polymers according to an embodiment of the present disclosure.
Fig. 4 is a diagram for illustrating a measurement method for a hydrogen peroxide concentration using an aggregate of hydrophilic polymers according to an embodiment of the present disclosure.
Fig. 5 is a diagram for illustrating a measurement method for a glucose concentration using an aggregate of hydrophilic polymers according to an embodiment of the present disclosure.

DESCRIPTION OF THE EMBODIMENTS

The present disclosure is described in more detail below.

In the present disclosure, the term "detection" refers to the determination of not only the presence or absence of the target substance but also the content, concentration or activity thereof. The term is used in a sense including both qualitative and quantitative cases, and may also be referred to as "measurement".

(Degree of Fluorescence Polarization determined by Fluorescence Polarization Measurement)

Fluorescence polarization measurement is used to analyze the mobility of fluorescent molecules in a solution. The principle of the fluorescence polarization measurement is described by taking the case of a luminescent particle of the present disclosure as an example. When a luminescent particle (the particle includes a luminescent molecule exhibiting fluorescence polarization) in a liquid is excited by plane-polarized light, the particle emits polarized fluorescence in the same plane. However, when the luminescent particle rotates by Brownian motion during the excited state, the particle emits fluorescence to a plane different from the excitation plane, and thus its fluorescence polarization is eliminated. In other words, the degree of fluorescence polarization represents the degree of rotational motion of the luminescent particle from being excited to emitting fluorescence.

A method of detecting a target substance according to an embodiment of the present disclosure is a method that utilizes the measurement of the mobility of a luminescent particle in a liquid sample. When the luminescent particle in a liquid is dispersed singly in a solution, the particle rotates vigorously by its Brownian motion, and thus exhibits a low degree of fluorescence polarization. Meanwhile, when the luminescent particle is bonded to another carrier having a large size, the Brownian motion in the solution decreases, and the degree of fluorescence polarization increases. Accordingly, in the fluorescence polarization measurement, the mobility of the luminescent particle in the solution is analyzed by using a change in degree of fluorescence polarization as an indicator. The degree of fluorescence polarization may be indicated by milli P (hereinafter abbreviated as "mp"), which represents a change in plane polarization.

The change in mobility of the luminescent particle may be detected as a change in degree of fluorescence polarization, that is, may be utilized in the detection of various target substances in a liquid sample by controlling the mobility.

In this embodiment, it has been described that the evaluation of the mobility of the luminescent particle may be evaluated by using the change in degree of fluorescence polarization as an indicator. In the present disclosure, however, the evaluation of the mobility of the luminescent particle only needs to be performed by using a change in value related to fluorescence anisotropy as an indicator, and polarization anisotropy may be used.

(Method of detecting Target Substance)

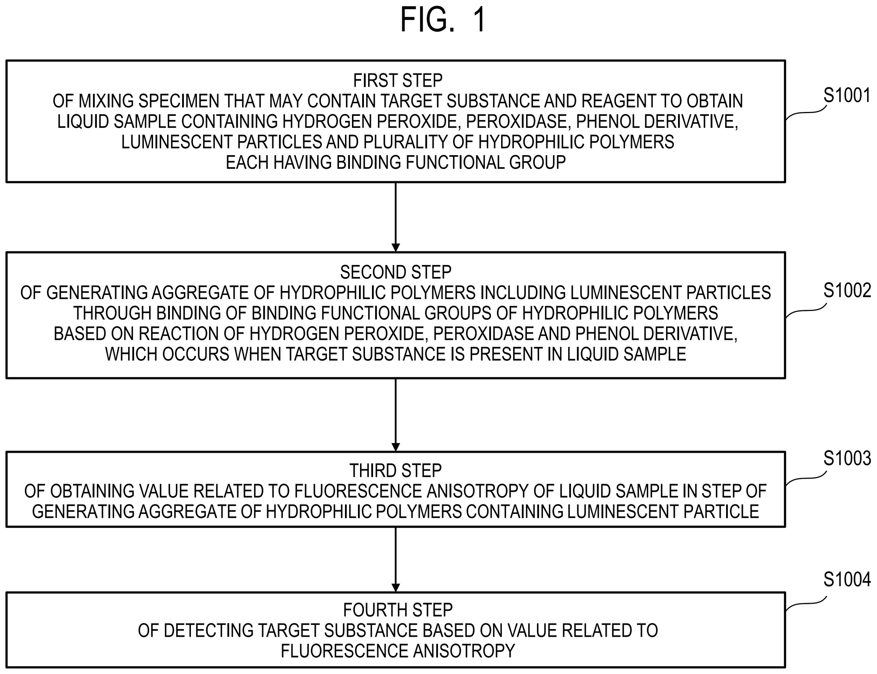

The method of detecting a target substance of this embodiment is a method of detecting a target substance related to an enzyme reaction, the method including the following steps (1) to (3). Here, at the time of the detection of the target substance, the method may include a step (4) of detecting the target substance using a value related to fluorescence anisotropy obtained in the step (3), as illustrated in Fig. 1:

(1) a first step (S1001) of mixing a specimen that may contain a target substance and a reagent to obtain a liquid sample containing hydrogen peroxide, a peroxidase, a phenol derivative, a luminescent particle and a plurality of hydrophilic polymers each having a binding functional group;
(2) a second step (S1002) of generating an aggregate of the hydrophilic polymers including the luminescent particle through the binding of the binding functional groups of the hydrophilic polymers based on a reaction of the hydrogen peroxide, the peroxidase and the phenol derivative, which occurs when the target substance is present in the liquid sample;
(3) a third step (S1003) of obtaining a value related to fluorescent anisotropy of the liquid sample in the step of generating the aggregate of the hydrophilic polymers containing the luminescent particle; and
(4) a fourth step (S1004) of detecting the target substance based on the value related to fluorescence anisotropy of the liquid sample.

(Measurement Principle 1: Measurement of Peroxidase Activity)

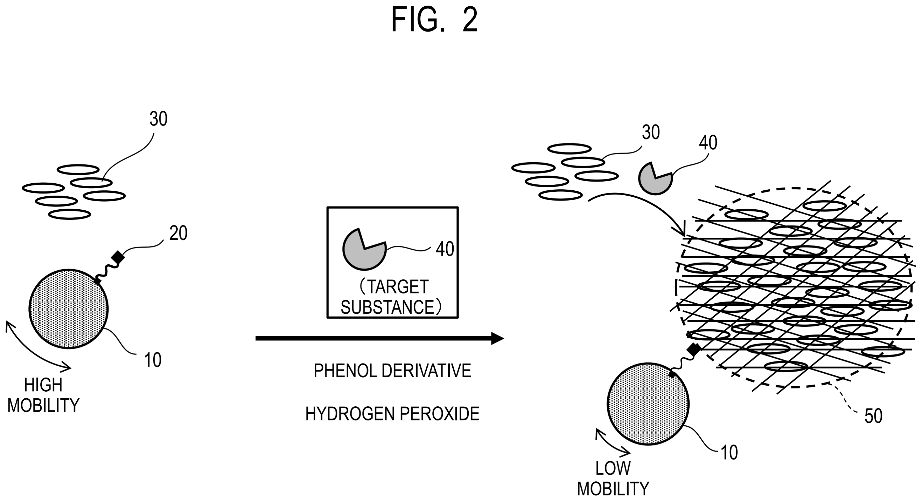

The measurement principle of the present disclosure is described with reference to Fig. 2 by a highly sensitive measurement method for the activity of a peroxidase, which is an example of an oxidoreductase, using the crosslinking of hydrophilic polymers.

A peroxidase 40, which is a target substance, can form an aggregate 50 of the hydrophilic polymers 30 (may also be referred to as "polymer crosslinked body") through the crosslinking of a plurality of hydrophilic polymers 30 each having a binding functional group (functional group having a binding ability) in the presence of specified amounts of hydrogen peroxide and a phenol derivative (including phenol). The formed aggregate 50 of the hydrophilic polymers 30 grows to a suitable size. Here, sodium alginate having a thiol group introduced thereinto is used as an example of the hydrophilic polymer 30 having a binding functional group. A luminescent particle 10 is allowed to coexist in the solution as a probe molecule for fluorescence polarization measurement. When the luminescent particle 10 also has, for example, a thiol group introduced as a binding functional group 20, a conjugate is formed between the aggregate 50 of the hydrophilic polymers 30 each formed of alginic acid and the luminescent particle 10 based on the binding of the thiol groups to each other. The luminescent particle 10 before the addition of the peroxidase 40 is dispersed in the solution and has high mobility. However, when the peroxidase 40 is added, the aggregate 50 of the hydrophilic polymers 30 is generated in accordance with its activity and binds to the luminescent particle 10 so that the mobility of the luminescent particle 10 is greatly reduced. The decrease in mobility is observed as an increase in value related to fluorescence anisotropy of the luminescent particle 10 (hereinafter described by using the degree of fluorescence polarization as an example of the value related to fluorescence anisotropy). Accordingly, the activity of the peroxidase 40 in the specimen may be determined by measuring the degree of fluorescence polarization of the luminescent particle 10. For example, the peroxidase activity of the specimen may be determined from the measurement result of the degree of fluorescence polarization of the solution based on a calibration curve for a degree of fluorescence polarization and activity obtained in advance by using a peroxidase having known activity.

(Measurement Principle 2: Measurement of Glucose Oxidase Activity)

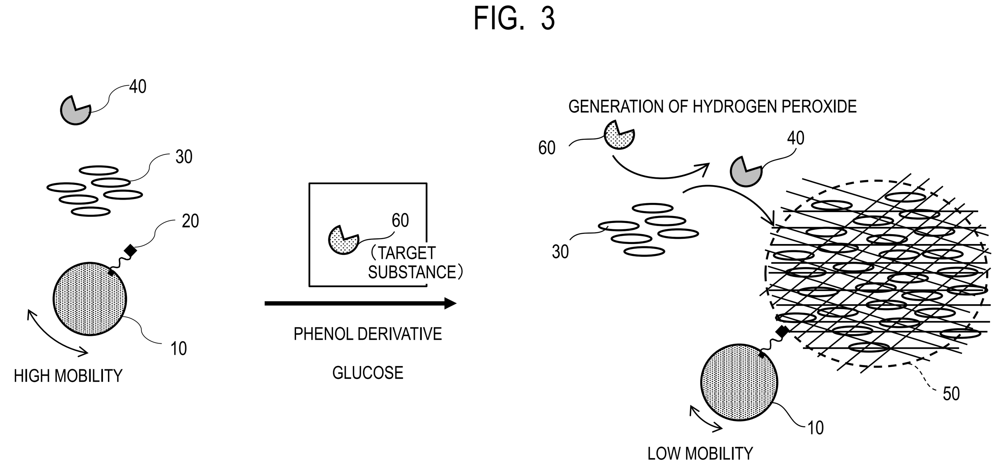

In addition to the peroxidase, as another substance related to a peroxidase enzyme reaction, the enzyme activity of an oxidase may be measured by a similar method. For example, description is made by taking glucose oxidase as an example (Fig. 3). The measurement of the glucose oxidase activity of the present disclosure utilizes a cascade reaction of two kinds of enzymes, a glucose oxidase 60 and the peroxidase 40. The glucose oxidase 60, which is the target substance, can rapidly crosslink a plurality of the hydrophilic polymers 30 each having a binding functional group in the presence of specified amounts of glucose, the peroxidase 40 and a phenol derivative to form the aggregate 50. This principle is the same as Measurement Principle 1 used for the formation of the aggregate 50 of the hydrophilic polymers 30 each formed of alginic acid by the peroxidase 40 described above except that glucose is converted into hydrogen peroxide depending on the activity of the glucose oxidase 60. The hydrogen peroxide activates the peroxidase 40, and the aggregate 50 of the hydrophilic polymers 30 is generated. In the same manner as in Measurement Principle 1 described above, by allowing the luminescent particle 10 to coexist in the solution as a probe molecule for fluorescence polarization measurement, the mobility of the luminescent particle 10 changes depending on the activity of the glucose oxidase 60. The glucose oxidase activity of the specimen may be determined from the measurement result of the degree of fluorescence polarization of the solution based on a calibration curve for a degree of fluorescence polarization and activity obtained in advance by using glucose oxidase having known activity.

(Measurement Principle 3: Measurement of Hydrogen Peroxide Concentration)

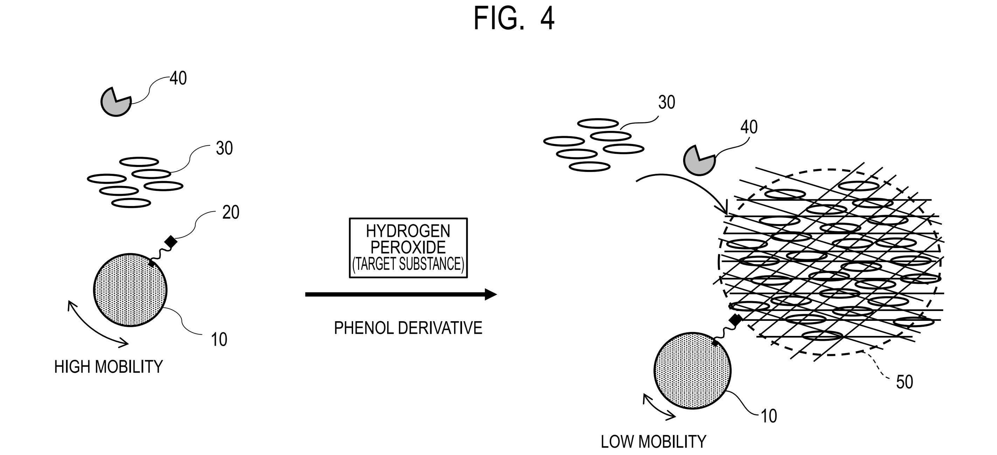

In the present disclosure, the concentration of hydrogen peroxide, which is an example of an enzyme reaction-related substance, may also be measured by a similar method (Fig. 4). For example, the hydrogen peroxide, which is the target substance, can rapidly form the aggregate 50 of the hydrophilic polymers 30 in the presence of specified amounts of the peroxidase 40 and a phenol derivative. The luminescent particle 10 is allowed to coexist in the solution as a probe molecule for fluorescence polarization measurement. The activity of the peroxidase 40 changes depending on the amount of the hydrogen peroxide, which is the target substance, and by measuring the generation of the aggregate 50 of the hydrophilic polymers 30 and the change in mobility of the luminescent particle 10 associated with the change by the fluorescence polarization measurement, it becomes possible to measure the hydrogen peroxide concentration. The hydrogen peroxide concentration may be determined from the measurement result of the degree of fluorescence polarization of the solution based on a calibration curve for a degree of fluorescence polarization and a hydrogen peroxide concentration obtained in advance by using hydrogen peroxide having a known concentration.

(Measurement Principle 4: Measurement of Glucose Concentration)

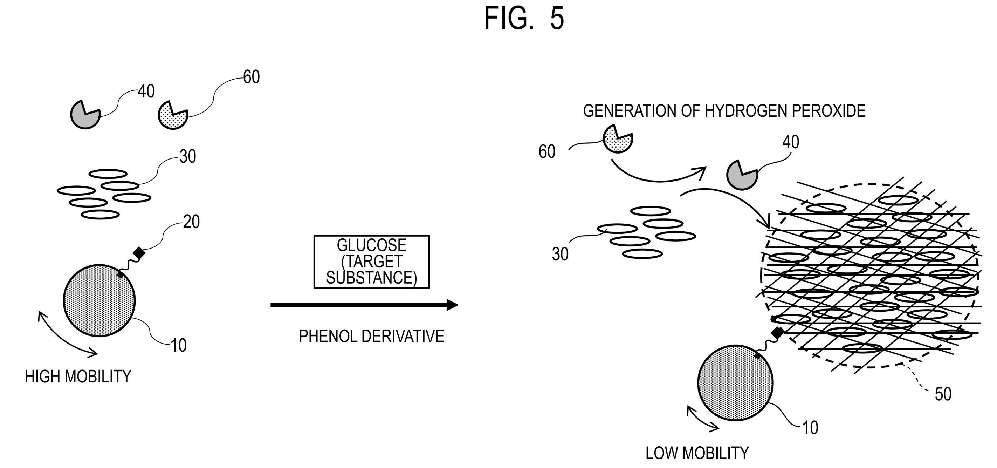

In the present disclosure, the concentration of glucose, which is an example of an enzyme reaction-related substance, may also be measured by a similar method (Fig. 5). For example, glucose, which is the target substance, can rapidly form the aggregate 50 of the hydrophilic polymers 30 (form the hydrophilic polymers into gel) in the presence of specified amounts of the glucose oxidase 60, the peroxidase 40 and a phenol derivative. In the same manner as in the measurement principle of the glucose oxidase activity described in Measurement Principle 2, the glucose concentration may be measured by utilizing the cascade reaction of the glucose oxidase 60 and the peroxidase 40. The luminescent particle 10 is caused to coexist in the solution as a probe molecule for fluorescence polarization measurement. By measuring the generation of the aggregate 50 of the hydrophilic polymers 30 and the change in mobility of the luminescent particle 10 associated with the change in activity of the peroxidase 40 depending on the amount of the glucose, which is the target substance, by the fluorescence polarization measurement, it becomes possible to measure the glucose concentration. The glucose concentration may be determined from the measurement result of the degree of fluorescence polarization of the solution based on a calibration curve for a degree of fluorescence polarization and a glucose concentration obtained in advance by using glucose having a known concentration.

(Measurement Principle 5: Measurement of Phenol Derivative Concentration)

In the present disclosure, the concentration of a phenol derivative, which is an example of an enzyme reaction-related substance, may also be measured by a similar method. For example, the phenol derivative, which is the target substance, can rapidly form the aggregate 50 of the hydrophilic polymers 30 by binding the binding functional groups of the hydrophilic polymers 30 in the presence of specified amounts of the peroxidase 40 and hydrogen peroxide. The luminescent particle 10 is allowed to coexist in the solution as a probe molecule for fluorescence polarization measurement. At this time, the luminescent particle 10 is trapped to form the aggregate 50 of the hydrophilic polymers 30. The degree (e.g., size) of the generation of the aggregate 50 of the hydrophilic polymers 30 by the peroxidase 40 changes depending on the amount of the phenol derivative, which is the target substance, and by measuring the change in mobility of the luminescent particle 10 associated with the change by the fluorescence polarization measurement, it becomes possible to measure the phenol derivative concentration. The phenol derivative concentration may be determined from the measurement result of the degree of fluorescence polarization of the solution based on a calibration curve for a degree of fluorescence polarization and a phenol derivative concentration obtained in advance by using a phenol derivative having a known concentration.

Thus, by forming the aggregate 50 of the hydrophilic polymers 30 using an enzyme reaction, it becomes possible to measure the enzyme reaction-related substance, which is the target substance, with high sensitivity and rapidly even when the amount of the substance is small or when the amount of contaminants is large.

(Specific Description of Measurement Method)

An example of the present disclosure includes the following steps:

(1) a first step of mixing a specimen that may contain a target substance and a reagent to obtain a liquid sample containing hydrogen peroxide, a peroxidase, a phenol derivative, a luminescent particle and a plurality of hydrophilic polymers each having a binding functional group;
(2) a second step of generating the aggregate of the hydrophilic polymers including the luminescent particle through binding of the binding functional groups of the hydrophilic polymers based on a reaction of the hydrogen peroxide, the peroxidase and the phenol derivative, which occurs when the target substance is present in the liquid sample; and
(3) a third step of measuring a value related to fluorescence anisotropy of the liquid sample in the step of generating the aggregate of the hydrophilic polymers containing the luminescent particle.

The above-mentioned method of detecting a target substance may include a fourth step of detecting the target substance based on the value related to fluorescence anisotropy. The respective steps are described in more detail below.

(First Step)

In the present disclosure, in the first step, a specimen containing a target substance and a reagent are mixed to obtain a liquid sample containing hydrogen peroxide, a peroxidase, a phenol derivative, a luminescent particle and a plurality of hydrophilic polymers each having a first binding functional group.

(Second Step)

In the present disclosure, the second step is a step of forming an aggregate of the hydrophilic polymers including the luminescent particle by an enzyme reaction, which occurs when the target substance is present in the liquid sample. For example, the aggregate of the hydrophilic polymers including the luminescent particle is obtained by reactions of an oxidoreductase, which is the target substance, the phenol derivative, the hydrogen peroxide, the luminescent particle and the hydrophilic polymers each having a binding functional group. Here, the order of the reactions is not particularly limited. That is, the oxidoreductase, which is the target substance, the phenol derivative, the hydrophilic polymer, the hydrogen peroxide and the luminescent particle may be simultaneously subjected to a reaction to obtain the aggregate of the hydrophilic polymers including luminescent particle. Alternatively, the oxidoreductase, which is the target substance, the phenol derivative, the hydrophilic polymers each having a binding functional group and the hydrogen peroxide may be first subjected to a reaction to generate the aggregate of the hydrophilic polymers, and then the luminescent particle may be subjected to a reaction with the aggregate of the hydrophilic polymers to obtain the aggregate of the hydrophilic polymers including the luminescent particle. In addition, the aggregate of the hydrophilic polymers including the luminescent particle may be a conjugate of the luminescent particle and the aggregate of the hydrophilic polymers.

When the target substance is hydrogen peroxide, which is an example of an enzyme reaction-related substance, the hydrogen peroxide, which is the target substance, is subjected to a reaction with a solution containing a peroxidase, a phenol derivative, hydrophilic polymers each having a binding functional group and a luminescent particle to form an aggregate of the hydrophilic polymers including the luminescent particle. Here, the order of the reactions is not particularly limited, but it is preferrable that the hydrogen peroxide, which is the target substance, be added last because the aggregate of the hydrophilic polymers is generated with the hydrogen peroxide as a trigger. In addition, the "aggregate of the hydrophilic polymers including the luminescent particle" encompasses both of: the case in which the aggregate of the hydrophilic polymer and the luminescent particle are bonded, for example, the case in which the binding functional group (first binding functional group) of each of the hydrophilic polymers of the aggregate of the hydrophilic polymers and the binding functional group (second binding functional group) of the luminescent particle are bonded; and the case in which the luminescent particle is trapped when the hydrophilic polymers form an aggregate and the aggregate of the hydrophilic polymers including the luminescent particle is formed.

When the target substance is glucose, which is an example of an enzyme reaction-related substance, the glucose, which is the target substance, may be subjected to a reaction with a solution containing glucose oxidase, a peroxidase, a phenol derivative, hydrophilic polymers each having a binding functional group and a luminescent particle to form an aggregate of the hydrophilic polymers including the luminescent particle. Here, the order of the reactions is not particularly limited, but it is preferrable that the glucose, which is the target substance, be added last because the aggregate of the hydrophilic polymers is generated with the glucose as a trigger.

When the target substance is a phenol derivative, which is an example of an enzyme reaction-related substance, the phenol derivative, which is the target substance, and hydrogen peroxide are subjected to a reaction with a solution containing a peroxidase, hydrophilic polymers each having a binding functional group and a luminescent particle to form an aggregate of the hydrophilic polymers including the luminescent particle. Here, the order of the reactions is not particularly limited, but it is preferrable that the hydrogen peroxide be added last because the aggregate of the hydrophilic polymers is generated with the hydrogen peroxide as a trigger.

(Liquid Sample)

The liquid sample that may be used in the present disclosure may be any liquid (also referred to as "specimen") that contains a component for measuring the activity or concentration of the target substance and also contains the target substance.

In the present disclosure, the target substance is an enzyme reaction-related substance.

The specimen is, for example, a body fluid, such as blood, urine or saliva, containing the target substance, a buffer solution containing the target substance, a culture liquid or tissue extract of cells or microorganisms containing the target substance, drinking water containing the target substance, river water containing the target substance or a waste liquid containing the target substance.

The specimen may contain hydrogen peroxide. The hydrogen peroxide promotes the reaction of a peroxidase, but various kinds of measurement may be performed by appropriately adjusting the concentrations and blending ratios of the peroxidase, a phenol derivative, a luminescent particle and the like and conditions for fluorescence polarization measurement.

(Oxidoreductase)

In the present disclosure, an oxidoreductase is preferrable as an enzyme to be used in the reaction. The oxidoreductase is an enzyme that catalyzes a redox reaction. Examples of the oxidoreductase include an oxidase, a dehydrogenase, a reductase and an oxygenase. Specific examples thereof include a peroxidase, various oxidases, catalase, glucose dehydrogenase, lactate dehydrogenase, pyruvate dehydrogenase, alcohol dehydrogenase and thioredoxin reductase. Further, examples of the peroxidase include horseradish peroxidase (HRP), myeloperoxidase, cytochrome P450 and manganese peroxidase. Examples of the oxidase include glucose oxidase, lactate oxidase, pyruvate oxidase, cholesterol oxidase, alcohol oxidase, amino acid oxidase and choline oxidase.

(Enzyme Reaction-related Substance)

The enzyme reaction-related substance in the present disclosure is a substance related to a peroxidase enzyme reaction, and may be any substance whose amount or activity may be measured by the measurement method of the present disclosure. That is, in such case, the enzyme reaction-related substance of a peroxidase may be any substance that affects the activity of the peroxidase. For example, the enzyme reaction-related substance may be a substance that generates a peroxidase by an enzyme reaction or a substance that causes a peroxidase enzyme reaction. More specifically, the enzyme reaction-related substance is a substrate for an oxidoreductase, hydrogen peroxide or a phenol derivative. The amount or activity of such enzyme reaction-related substance changes the degree of fluorescence polarization obtained by the fluorescence polarization measurement of the present disclosure. Examples of the substrate for an oxidoreductase include glucose, lactic acid, pyruvic acid and cholesterol.

(Luminescent Particle)

The luminescent particle to be used in the present disclosure may be any luminescent particle which is a nanoparticle containing a luminescent substance and whose degree of fluorescence polarization for luminescence may be measured by the fluorescence polarization measurement. In addition, the luminescent particle according to the present disclosure may bind to an aggregate of hydrophilic polymers generated from hydrophilic polymers each having a binding functional group (which may be referred to as "first binding functional group") or may be included when the aggregate of the hydrophilic polymers is formed, but the luminescent particle is preferably bonded to the aggregate of the hydrophilic polymers. The mode of binding when the luminescent particle and the aggregate of the hydrophilic polymers bind to each other may be a chemical bond, such as a covalent bond, an ionic bond or a coordinate bond, or physical adsorption. When the mode of binding is a chemical bond, the luminescent particle preferably has a binding functional group (which may be referred to as "second binding functional group"), such as a thiol group, an amino group, a carboxy group or a maleimide group, as described later. Of those, a thiol group is particularly preferable because the reaction proceeds mildly and rapidly.

The luminescent particle of the present disclosure is a nanoparticle, and the average particle diameter that is the average of the diameters of the particles is a diameter of 1 nm or more to 1,000 nm or less, preferably 25 nm or more to 500 nm or less, and the average particle diameter is more preferably 50 nm or more to 300 nm or less. When the average particle diameter is more than 500 nm, the degree of fluorescence polarization of the luminescent particle itself before an interaction with the hydrophilic polymer may increase, and the amount of change in degree of fluorescence polarization after the reaction (after the interaction with the hydrophilic polymer) may decrease. In addition, when the average particle diameter is less than 25 nm, the content of the luminescent substance in the nanoparticle decreases, and the luminescence intensity per particle decreases. As a result, the measurement sensitivity and accuracy may decrease, and hence a luminescent particle of 50 nm or more is preferrable. The size of the luminescent particle may be examined by dynamic light scattering (DLS) measurement.

As a particle material (matrix material) for the luminescent particle, for example, a nanoparticle of a synthetic polymer, such as polystyrene or polymethacrylate, an inorganic nanoparticle, such as silica, titanium oxide or iron oxide, or a natural polymer, such as dextran or casein, may be used. Of those, a synthetic polymer is preferrable as the particle material for the luminescent particle according to the present disclosure because the synthetic polymer has a specific gravity that allows dispersion in water, is stable, and has high productivity, and a polystyrene particle excellent in particle size controllability is particularly preferrable.

In addition, it is preferrable that the surface of the particle be hydrophilized. This is because by hydrophilizing the surface of the particle, non-specific aggregation between the particles can be suppressed, and non-specific adsorption of the particle to a measurement container (e.g., plastic cuvette or quartz glass) can also be suppressed. In the measurement method using the fluorescence polarization measurement of the present disclosure, a change in mobility of the luminescent particle irrespective of the activity or amount of an enzyme or the concentration of an enzyme reaction-related substance causes a decrease in accuracy or precision of the measurement of the target substance. Accordingly, it is required to avoid non-specific aggregation between the particles and non-specific adsorption of the particle to the container. For this reason, it is preferrable that the surface of the particle be coated with a hydrophilic polymer. For example, a polymer, such as polyvinylpyrrolidone, polyethylene glycol, polyhydroxymethacrylate, dextran or bovine serum albumin, is suitably used. In addition, as described above, the luminescent particle preferably binds to the aggregate of the hydrophilic polymers. In order to promote the binding between the luminescent particle and the aggregate of the hydrophilic polymers, when the binding functional group of each of the hydrophilic polymers is defined as a first binding functional group, a second binding functional group may be appropriately introduced into the surface of the luminescent particle. The first binding functional group and the second binding functional group may be the same (kind of) functional group, or may be different kinds of functional groups. For example, when the second binding functional group, such as a thiol group, an amino group, a carboxy group or a maleimide group, is present on the surface of the luminescent particle, the binding between the luminescent particle and the aggregate of the hydrophilic polymers each having the first binding functional group, such as a thiol group, a carboxy group, an amino group, can be promoted. This is because an electrostatic bond, a disulfide bond, a bond between a thiol group and a maleimide group or the like occurs between the luminescent particle and the aggregate of the hydrophilic polymers. Accordingly, as a preferrable example of the surface of the luminescent particle, one that is coated with a hydrophilic polymer such as polyvinylpyrrolidone and further has a thiol group introduced is preferrable.

The luminescent particle to be used in the present disclosure may be configured such that luminescent particle does not specifically bind to the target substance. That is, the luminescent particle does not serve as a substrate for an enzyme, which is an example of the target substance of the present disclosure. By adopting such mode, the luminescent particle can function independently as a probe in the fluorescence polarization measurement. The ability to function independently has the advantage in that, for example, the optimal conditions for the luminescent particle or the enzyme can be set separately and hence the design of measurement conditions is facilitated. An immunoassay using an antibody-bound luminescent particle in which an antibody against an enzyme is bound to the luminescent particle is known for detecting an enzyme as the target substance, but the measurement of a trace amount of an enzyme depends on the binding affinity of an antibody, and the construction of a high-sensitivity assay involves complicated work. In addition, in an immunoassay, it is extremely difficult to evaluate the activity of an enzyme. Meanwhile, in the method of the present disclosure, by loading sufficient amounts of a substrate and a luminescent particle, it becomes possible to increase the sensitivity of the measurement of the amount or activity of an enzyme depending on a reaction time.

The luminescent particle to be used in the present disclosure is characterized by containing a luminescent substance. Here, the luminescent substance may be any substance that can be incorporated into a particle and can be measured by fluorescence polarization measurement. The luminescent substance may be incorporated into the surface of the particle or the inside of the particle, and is preferably incorporated into the inside of the particle. This is because an interaction between the luminescent substance and contaminants or water molecules in a solution may occur on the surface of the particle to affect the dispersibility of the luminescent particle and fluorescence polarization measurement.

Examples of the luminescent substance include a fluorescent dye, such as a fluorescein derivative, a rhodamine derivative or a cyanine derivative, and a rare earth luminescent complex, such as a europium complex or a terbium complex. The europium complex is suitable as the luminescent substance to be incorporated into the luminescent particle according to the present disclosure because of the following features: its emission wavelength and intensity are less liable to be affected by the surroundings and its emission lifetime is long.

Specific examples of the europium complex include [tris(2-thenoyltrifluoroacetone)bis(triphenylphosphineoxide)europium(III)], [tris(2-thenoyltrifluoroacetone)(triphenylphosphineoxide)(dibenzylsulfoxide)europium(III)] and [tris(2-thenoyltrifluoroacetone)(phenanthroline)europium(III)].

It is preferable that the content of the luminescent substance in the luminescent particle be higher. This is because the luminescence intensity per particle increases, and as a result, the increase can contribute to higher sensitivity or improved measurement accuracy for measuring the activity or amount of an enzyme or the concentration of an enzyme reaction-related substance of the present disclosure. It is preferable that the content of the rare earth luminescent complex in the luminescent particle be, for example, 0.001 g or more of the rare earth luminescent complex per 1 g of the particle. With this content, the luminescence intensity per particle is sufficiently high and stable luminescence can be obtained. In addition, the content of the luminescent substance may be measured by elemental analysis using the specific element of the luminescent substance as an indicator, and for example, the content of the rare earth luminescent complex may be calculated from the quantification of the rare earth luminescent complex by inductively coupled plasma (ICP) emission measurement.

(Phenol Derivative)

The phenol derivative to be used in the present disclosure is not particularly limited as long as the phenol derivative is a phenol compound having a phenolic hydroxy group and is capable of generating a phenoxy radical by the action of a peroxidase. An example thereof is a low-molecular-weight phenol compound having at least one phenolic hydroxy group and having a molecular weight of 1,000 or less. The phenol derivative is preferably a low-molecular-weight phenol compound having 1 to 6 phenolic hydroxy groups and having a molecular weight of 500 or less. Examples thereof include phenol, catechol, resorcinol, hydroquinone, tyramine and a hydrochloride thereof, serotonin, N-glycyl-L-tyrosine (Gly-Tyr), 3-(4-hydroxyphenyl)propionic acid, methyl 3-(4-hydroxyphenyl)propionate, 4-hydroxyphenylacetic acid, 3-hydroxyphenylacetic acid, p-coumaric acid, caffeic acid, dopamine, 6-hydroxydopamine and norepinephrine. From the viewpoints of water solubility and reaction stability, tyramine, tyramine hydrochloride, phenol, glycyl-L-tyrosine, resorcinol and serotonin are preferably used. In the present disclosure, the phenol derivative may be referred to as phenols.

(Hydrophilic Polymer having Binding Functional Group)

In the present disclosure, a hydrophilic polymer having a binding functional group is a molecule that serves as a raw material for generating an aggregate of hydrophilic polymers, and includes a skeleton molecule and a binding functional group. In consideration of the fact that the aggregate of the hydrophilic polymers is a gel-like substance, the hydrophilic polymer having a binding functional group may also be referred to as "gel precursor." It is appropriate that the hydrophilic polymer having a binding functional group be a substance having a molecular weight in the range of from about 1,000 to about 5,000,000, more preferably from about 10,000 to about 1,000,000. In an example of the measurement method of the present disclosure, it is required to incorporate the luminescent particle into the generated aggregate of the hydrophilic polymers to greatly reduce the mobility of the luminescent particle. Meanwhile, before the measurement of the target substance, the molecular weight of the hydrophilic polymer having a binding functional group is preferably small from the viewpoint of handling the solution. In addition, it is preferrable that the hydrophilic polymer be free from hindering the measurement of the target substance. The hydrophilic polymer preferably has a smaller molecular weight, but is required to have a large number of binding functional groups, such as a phenol group and a thiol group, required for the formation of the aggregate of the hydrophilic polymers. It is required that the hydrophilic polymer be water-soluble, have no interaction with an enzyme, and not be too viscous. From those requirements, alginic acid, hyaluronic acid, dextran, albumin, gelatin, polyethylene glycol and the like are given as examples of the skeleton molecule of the hydrophilic polymer.

In the present disclosure, HRP may be used to rapidly crosslink a plurality of hydrophilic polymers each having a binding functional group to form an aggregate of the hydrophilic polymers. A substrate for a HRP-catalyzed reaction is known to be a phenol group or a thiol group. Accordingly, the hydrophilic polymer having a binding functional group of the present disclosure is a water-soluble molecule having a large number of those binding functional groups, and is a molecule that, when subjected to an oxidation reaction by HRP, can form an aggregate of hydrophilic polymers by crosslinking the hydrophilic polymers through the binding (covalent bonding) of the binding functional groups of different hydrophilic polymers to each other (that is, can form gel). At this time, the binding of the binding functional groups may be the binding of different kinds of binding functional groups, or the binding of the same kind of binding functional groups. That is, the aggregate of the hydrophilic polymers may be formed by the binding of different kinds of binding functional groups when the hydrophilic polymers have different kinds of binding functional groups, or the aggregate of the hydrophilic polymers may be formed by the binding of the same kind of binding functional groups.

Examples of the hydrophilic polymer having a phenol group or a thiol group as the binding functional group include phenol group-introduced sodium alginate, phenol group-introduced hyaluronic acid, phenol group-introduced gelatin, phenol group-introduced albumin, thiol group-introduced sodium alginate, thiol group-introduced hyaluronic acid, thiol group-introduced gelatin and thiol group-introduced albumin.

A method of introducing the phenol group into a water-soluble molecule may introduce, for example, tyramine into the carboxy group of sodium alginate with a condensing agent. Instead of tyramine, tyrosine, which is an amino acid, may be used. A method of introducing the thiol group into a water-soluble molecule may introduce, for example, aminoethanethiol into the carboxy group of sodium alginate with a condensing agent. Instead of aminoethanethiol, cysteine, which is an amino acid, may be used. In addition, the thiol group may be generated by reducing an intramolecular disulfide bond by reducing albumin.

In the case of a phenol group-introduced hydrophilic polymer, HRP catalyzes an oxidative coupling reaction of the phenol groups in the presence of hydrogen peroxide so that the phenol group-introduced hydrophilic polymers can form gel (form an aggregate of the hydrophilic polymers). In this case, it is preferrable that a phenol group, which is the same functional group as the first binding functional group, be similarly introduced as a second binding functional group into the surface of the luminescent particle.

The size of the aggregate of the hydrophilic polymers for greatly changing the degree of fluorescence polarization of the luminescent particle in accordance with the enzyme activity may be observed by dynamic light scattering (DLS) measurement. For example, when the average particle diameter of the luminescent particles is about 100 nm, the aggregate of the hydrophilic polymers is preferably about 1,000 nm in size. The binding of the luminescent particle to the aggregate of the hydrophilic polymers, which is about 10 times as large as the average particle diameter thereof, results in a volume change of about 1,000-fold to greatly reduce the mobility of the luminescent particle and make it possible to obtain a large signal change with the fluorescence polarization measurement. The increase in average particle diameter is preferably at least 1.5 times or more, preferably 5 times or more, particularly preferably 10 times or more.

(Hydrogen Peroxide)

In the present disclosure, hydrogen peroxide is used for the crosslinking reaction of hydrophilic polymers each having a binding functional group by HRP. The mechanism of a gel-forming reaction by a peroxidase utilized in the method of the present disclosure is as disclosed in U.S. Patent Application Publication No. 2017/0239356 (International Publication No. WO2015/159995). A radical transfer reaction from a phenoxy radical generated in a reaction solution to the thiol group occurs by the action of the phenol derivative on HRP, and a thiol radical rapidly undergoes a disulfide bond reaction with another thiol group. In addition, it has been reported that hydrogen peroxide is generated during the disulfide bond reaction, and the catalytic cycle of HRP proceeds spontaneously without the addition of hydrogen peroxide from the outside. As a result, it has been reported that the oxidation of SH groups (disulfide bond reaction) by HRP is promoted under the conditions of a short period of time and a low HRP concentration. However, as described later in Example 1 and Comparative Example 2, it has been found that the addition of hydrogen peroxide from the outside is essential for the measurement by the fluorescence polarization measurement of the present disclosure. This is because the present disclosure aims at rapid measurement, and specifically, it is required to generate an aggregate of hydrophilic polymers for greatly changing the degree of fluorescence polarization of the luminescent particle as a probe in a reaction time of about several minutes. Under the conditions that hydrogen peroxide is not used, which is shown in the related art (Comparative Example 2), a sufficient amount of gel could not be generated to change the degree of fluorescence polarization of the luminescent particle. Accordingly, it is conceived that the addition of hydrogen peroxide is essential for the measurement method of the present disclosure to be established. Hydrogen peroxide is a peroxide and functions as an oxidizing agent.

When the activity of glucose oxidase is measured, the addition of hydrogen peroxide from the outside is not essential because hydrogen peroxide is generated by the action of glucose oxidase, but an appropriate amount of hydrogen peroxide may be added to accelerate the gel-forming reaction and obtain a large signal.

(Third Step)

In the present disclosure, the third step is a step of obtaining a value related to fluorescence anisotropy of the liquid sample in the step of generating the aggregate of the hydrophilic polymers containing the luminescent particle described above. The fluorescence polarization measurement of obtaining the value related to fluorescence anisotropy may be performed by a known method, and various commercially available measurement apparatus may be used for that purpose. The fluorescence polarization measurement is also called fluorescence depolarization measurement, but in the present specification, the fluorescence polarization measurement and the fluorescence depolarization measurement are synonymous. Herein, the value related to fluorescence anisotropy is specifically the degree of fluorescence polarization, but may be a value indicating fluorescence anisotropy determined by calculation from the degree of fluorescence polarization, or may be a value that becomes a value indicating the degree of fluorescence polarization by calculation. The degree of fluorescence polarization (p) and the fluorescence anisotropy (r) have such a relationship as represented by the following formula (1).

$$P = 3r/(2+r) \ldots (1)$$

The degree of fluorescence polarization may be indicated by milli P (hereinafter abbreviated as "mp"), which shows the change in plane polarization, and is defined by the following formula (2).

$$mp = \{(I_H - I_V)/(I_H + I_V)\} \times 1{,}000 \ldots (2)$$

In the formula, $I_H$ and $I_V$ represent the intensities of fluorescence signals polarized in a plane parallel to and perpendicular to the polarization direction of excitation light, respectively.

In the present disclosure, an enzyme or an enzyme reaction-related substance, which is the target substance, may be subjected to fluorescence polarization measurement by using the mobility of a luminescent particle in a sample solution as an indicator. That is, as a result of the generation of an aggregate of hydrophilic polymers in the sample solution and the incorporation of the luminescent particle into the aggregate of the hydrophilic polymers (the luminescent particle binds to the aggregate of the hydrophilic polymers or the luminescent particle is trapped in the aggregate of the hydrophilic polymers) in accordance with the activity or amount of the enzyme or the amount of the enzyme reaction-related substance, the mobility of the luminescent particle decreases. A feature of the present disclosure is to measure the mobility of the luminescent particle by the fluorescence polarization measurement. In the fluorescence polarization measurement, the degree of fluorescence polarization (mp) may be used as an indicator.

A feature of the present disclosure is to use, as a probe, a luminescent particle containing a europium complex, which exhibits polarized luminescence, as a luminescent substance in the particle. A slight change in rotational movement of the luminescent particle in a liquid sample may be captured as a change in polarized luminescence characteristic. Specifically, when the mobility of the particles is greatly reduced by the fixation of the luminescent particle in a gel that is larger in size than that of the luminescent particle, the decrease in rotational Brownian motion of the particle can be captured with high sensitivity as a change in degree of fluorescence polarization.

The timing of obtaining the value related to the degree of fluorescence polarization may be set appropriately. For example, the value related to the degree of fluorescence polarization may be obtained after the second step. This mode is preferrable because the value related to the degree of fluorescence polarization only needs to be obtained when the reaction is finished, and the operation is simple. For example, the value related to the degree of fluorescence polarization may be obtained 5 minutes after the target substance is added to the solution.

Alternatively, the degree of fluorescence polarization of the sample solution may be measured before the reaction of the target substance (enzyme or enzyme reaction-related substance) in the second step, for example, at the stage where the first liquid sample is obtained, and the degree of fluorescence polarization may be set as an initial degree of fluorescence polarization (mp(0)), and then the reaction may proceed and a degree of fluorescence polarization (mp(t)) at a certain time point may be measured again after the binding of the luminescent particle to the generated aggregate of the hydrophilic polymers, or the degree of fluorescence polarization may be continuously measured at regular time intervals. That is, by measuring the change in degree of fluorescence polarization over time, the amount of change in degree of fluorescence polarization (Δmp) may be determined from the difference between the degree of fluorescence polarization (mp(t)) at a certain time point and the initial degree of fluorescence polarization (mp(0)). The change in degree of fluorescence polarization occurs rapidly, and for example, a sufficient Δmp can be obtained in from about 1 minute to about 10 minutes. A rate of change in degree of fluorescence polarization (dmp/dt) may also be determined.

Measurement conditions for the value related to the degree of fluorescence polarization are preferably, for example, as follows: in a liquid at a temperature of from 1°C to 50°C, the viscosity of the liquid is from 0.5 mPa·s to 50 mPa·s. When the luminescent particle is a luminescent particle containing a europium complex, the concentration of the luminescent particle is not limited as long as the luminescence of the luminescent particle can be detected, and the concentration of the luminescent particle may be appropriately selected in accordance with, for example, the kind of the target substance or the amount of hydrogen peroxide. It is preferable to measure the degree of fluorescence polarization in the range of from 0.0001 mg/mL to 1.0 mg/mL, but in order to ensure the luminescence intensity from the luminescent particle and avoid the influence of scattering of the luminescent particle, it is preferable to measure the degree of fluorescence polarization in the range of from 0.001 mg/mL to 0.1 mg/mL.

(Fourth Step)

In the present disclosure, the fourth step is a step of detecting the target substance in the liquid sample based on the value related to fluorescence anisotropy of the liquid sample obtained in the third step. More specifically, the fourth step is a step of associating a change in degree of fluorescence polarization with the activity or amount of an enzyme or the amount of an enzyme reaction-related substance.

In the third step, the degree of the decrease in mobility of the luminescent particle in response to the interaction with the aggregate of the hydrophilic polymers is detected as the value related to the degree of fluorescence polarization of the liquid sample. The degree of the decrease in mobility of the luminescent particle depends on the activity or amount of the oxidoreductase or the amount of the enzyme reaction-related substance in the liquid sample. Accordingly, by associating the value related to the degree of fluorescence polarization obtained in the third step with the activity or amount of the oxidoreductase or the amount of the enzyme reaction-related substance in the liquid sample, the activity or amount of the oxidoreductase or the amount of the enzyme reaction-related substance in the liquid sample can be measured. For example, a case in which the value related to the degree of fluorescence polarization obtained in the third step is large means that the luminescent particle strongly binds to the aggregate of the hydrophilic polymers, and this can be associated with high oxidoreductase activity.

The term "detection method" in the present disclosure includes not only quantitative measurement but also qualitative measurement (detection of the presence or absence of an enzyme or an enzyme reaction-related substance). Accordingly, the association of the value related to fluorescence anisotropy of the luminescent particles with the activity or amount of the oxidoreductase or the amount of the enzyme reaction-related substance in the fourth step may be quantitative or qualitative.

Quantitative association may be performed by, for example, determining a relational expression between enzyme activity and a value related to a degree of fluorescence polarization in advance using an enzyme solution having known enzyme activity, and then using this expression to determine the enzyme activity of the target substance from a value related to fluorescence anisotropy obtained by measuring the target substance. By performing such association, the enzyme activity in the liquid sample can be measured. The enzyme activity measured here may also be determined as an enzyme concentration, a mass, the number of molecules or the like. The value related to the degree of fluorescence polarization to be obtained may be the change in degree of fluorescence polarization of the liquid sample before and after the second step.

In the present disclosure, similarly, a relational expression between the concentration of hydrogen peroxide or glucose and a value related to a degree of fluorescence polarization may be determined in advance by using, for example, a hydrogen peroxide solution having a known concentration or a glucose solution having a known concentration. The concentration of the target substance can be determined from the degree of fluorescence polarization obtained by measuring the target substance.

In addition, in the fourth step, the change in value related to the degree of fluorescence polarization of the liquid sample may be handled relatively. That is, the activity or amount of an enzyme or the amount of an enzyme reaction-related substance may be associated relatively, and for example, the value of the degree of fluorescence polarization (mp) obtained by fluorescence polarization measurement may be used. For example, in applications for screening enzymes having high activity, screening may be performed simply based on the value of the degree of fluorescence polarization (mp) or the value of its amount of change ($\Delta$mp) for an enzyme population of the same concentration.

(Reagent for Inspection for detecting Target Substance using Measurement of Degree of Fluorescence Polarization)

According to the present disclosure, there may be provided a reagent for inspection as listed in any one of the following items (1) to (3), utilizing the above-mentioned measurement system.

(1) A reagent for inspection to be used in a method of measuring an enzyme reaction-related substance of a peroxidase in the present disclosure, the reagent including a luminescent particle, a phenol derivative, a hydrophilic polymer having a binding functional group and hydrogen peroxide. When the target substance is a peroxidase, the reagent may be a reagent including hydrogen peroxide, a phenol derivative, a luminescent particle and a hydrophilic polymer having a binding functional group. When the target substance is an oxidase, the reagent may be a reagent including a peroxidase, hydrogen peroxide, a phenol derivative, a luminescent particle and a hydrophilic polymer having a binding functional group. When the target substance is hydrogen peroxide, the reagent may be a reagent including a peroxidase, a phenol derivative, a luminescent particle and a hydrophilic polymer having a binding functional group. When the target substance is a substrate for an oxidase, the reagent may be a reagent including an oxidase, a peroxidase, a phenol derivative, a luminescent particle and a hydrophilic polymer having a binding functional group. When the target substance is a phenol derivative, the reagent may be a reagent including a peroxidase, hydrogen peroxide, a luminescent particle and a hydrophilic polymer having a binding functional group.

When the target substance is a glucose, the reagent may be a reagent including a glucose oxidase, a peroxidase, a luminescent particle, a phenol derivative and a hydrophilic polymer having a binding functional group.

The luminescent particle, the phenol derivative and the hydrophilic polymer having a binding functional group may be provided in a solution state, a dry state, a frozen state, a lyophilized state and the like. Hydrogen peroxide may be provided in a solution state. As a stabilizer for hydrogen peroxide, conventionally used salicylic acid or the like may be included.

The reagent for inspection of a target substance of the present disclosure may be a single reagent, or may be divided into a plurality of reagents, for example, a first liquid, a second liquid and a third liquid. From the viewpoint of the stability of each reagent, for example, the first liquid includes a luminescent particle, the second liquid includes a phenol derivative and a hydrophilic polymer having a binding functional group, and the third liquid includes hydrogen peroxide. In addition, the reagent for inspection of a target substance of the present disclosure may be divided into a solution state and a dry state, and the reagent in the dry state may be subjected to various kinds of measurement by being brought into a solution state with an attached dissolving liquid before the measurement.

(2) A reagent for inspection to be used in a method of measuring hydrogen peroxide that is a target substance according to the present disclosure, the reagent including an oxidoreductase, a luminescent particle, a phenol derivative and a hydrophilic polymer having a binding functional group.

The oxidoreductase, the luminescent particle, the phenol derivative and the hydrophilic polymer having a binding functional group may be provided in a solution state, a dry state, a frozen state, a lyophilized state and the like. In addition, the reagent for inspection of a target substance of the present disclosure may be divided into a plurality of reagents, for example, a first liquid, a second liquid and a third liquid. For example, the first liquid includes an oxidoreductase, the second liquid includes a luminescent particle, and the third liquid includes a phenol derivative and a hydrophilic polymer having a binding functional group.

(3) A reagent for inspection to be used in a method of measuring glucose that is a target substance according to the present disclosure, the reagent including glucose oxidase, a peroxidase, a luminescent particle, a phenol derivative and a hydrophilic polymer having a binding functional group.

The glucose oxidase, the peroxidase, the luminescent particle, the phenol derivative and the hydrophilic polymer having a binding functional group may be provided in a solution state, a dry state, a frozen state, a lyophilized state and the like. In addition, the reagent for inspection of a target substance of the present disclosure may be divided into a plurality of reagents, for example, a first liquid, a second liquid, a third liquid and a fourth liquid. For example, the first liquid includes glucose oxidase, the second liquid includes a peroxidase, the third liquid includes a luminescent particle, and the fourth liquid includes a phenol derivative and a hydrophilic polymer having a binding functional group.

[Examples]

The present disclosure is described in more detail below by way of Examples.

(1: Production of Thiol Group-introduced Luminescent Particles T1)

A luminescent particle to be used in a method of measuring the activity or amount of an enzyme or the amount of an enzyme reaction-related substance according to the present disclosure is an example in which the luminescent particle has a second binding functional group capable of binding to an aggregate of hydrophilic polymers on its surface. Here, a synthesis example of a luminescent particle having a thiol group as a second binding functional group on its surface is shown.

First, polyvinylpyrrolidone (PVP-K30: manufactured by Tokyo Chemical Industry Co., Ltd.) was dissolved in a 2-morpholinoethanesulfonic acid (MES) buffer solution (manufactured by Kishida Chemical Co., Ltd.) having a pH of 7 to prepare a solvent A. Subsequently, [tris(2-thenoyltrifluoroacetone)bis(triphenylphosphineoxide)europium(III)] (manufactured by Central Techno Corporation, hereinafter abbreviated as "Eu(TTA)$_3$(TPPO)$_2$") serving as a europium complex, a styrene monomer (manufactured by Kishida Chemical Co., Ltd.) and 3-methacryloxypropyltrimethoxysilane (manufactured by Tokyo Chemical Industry Co., Ltd., hereinafter abbreviated as "MPS") were mixed to prepare a reaction liquid B. The reaction liquid B was added into a four-necked flask containing the solvent A, and the mixture was stirred with a mechanical stirrer set to 300 rpm.

After 15 minutes of stirring under a nitrogen flow condition, the temperature of an oil bath that had been prepared was set to 70°C, and the nitrogen flow was performed for an additional 15 minutes. After the mixed liquid had been heated and stirred, an aqueous solution having dissolved therein potassium persulfate (hereinafter abbreviated as "KPS") (manufactured by Sigma-Aldrich) was added into the reaction solution, and emulsion polymerization was performed for 20 hours. After the polymerization reaction, the resultant suspension was subjected to ultrafiltration with about 4 L of ion-exchanged water through use of an ultrafiltration membrane having a molecular weight cutoff of 100K to wash the product, to thereby provide a dispersion of luminescent particles.

An aliquot of the dispersion of the luminescent particles obtained by the emulsion polymerization was taken and added to an aqueous solution having dissolved therein 1% by mass of Tween 20 (manufactured by Kishida Chemical Co., Ltd.). After 10 minutes of stirring, a silane coupling agent, X12-1135 (manufactured by Shin-Etsu Chemical Co., Ltd.), was added, and the mixture was stirred overnight (a mass ratio among the particles, pure water and X12-1135 loaded was 1:300:2). After the stirring, the dispersion was centrifuged, the supernatant was removed, and the precipitate was redispersed with pure water. The operations of centrifugation and redispersion were performed three or more times to wash the product. The precipitate after the washing was redispersed in pure water. Thus, luminescent particles 1 were obtained.

Next, a 0.25 mL aliquot of the particle dispersion of the luminescent particles 1 (particle concentration: 1.2% by mass) was taken, and the solvent was replaced with 1.6 mL of a MES buffer solution having a pH of 6.0. To the MES buffer solution in which the particles were dispersed, 1-[3-(dimethylamino)propyl]-3-ethylcarbodiimide and N-hydroxysulfosuccinimide sodium salt were added at 0.5% by mass, and the mixture was subjected to a reaction at 25°C for 1 hour. After the reaction, the dispersion was washed with a MES buffer solution having a pH of 5.0, 2-aminoethanethiol hydrochloride was added thereto, and the mixture was subjected to a reaction at 25°C for 2 hours to introduce 2-aminoethanethiol into the surface of each of the particles. After the introduction, the particles were washed with a Tris buffer solution having a pH of 8. After that, the particles were washed with a phosphate buffer solution to provide thiol group-modified luminescent particles having a concentration of 0.3% by mass (hereinafter referred to as "luminescent particles T1"). The aqueous solution containing the luminescent particles T1 is referred to as "luminescent particle T1 solution." The thiol-group-modified luminescent particles may also be referred to as "luminescent particles each having a second binding functional group."

The introduction of the thiol group into each of the particles was recognized by adding Ellman's reagent to the luminescent particle T1 solution, centrifuging the luminescent particle T1 solution, and then measuring the absorbance (410 nm) of the supernatant. The presence of the thiol group on the surface of each of the particles was able to be recognized by an increase in the absorbance as compared to that before the addition of Ellman's reagent. (2: Production of Thiol Group-introduced Sodium Alginate A1)

The hydrophilic polymer having a first binding functional group to be used in the method of measuring enzyme activity according to the present disclosure is a hydrophilic polymer having a first binding functional group that is crosslinked by an oxidoreductase to form an aggregate of hydrophilic polymers. Here, sodium alginate having a thiol group was synthesized.

First, sodium alginate (manufactured by FUJIFILM Wako Pure Chemical Corporation) was dissolved in ion-exchanged water to prepare a solution having a concentration of 1.0% by mass. 0.25 mL of the solution was added to 1.6 mL of a MES buffer solution having a pH of 6.0. 1-[3-(Dimethylamino)propyl]-3-ethylcarbodiimide was added to the resultant sodium alginate MES buffer solution so as to have a final concentration of 0.5% by mass. Further, 2-aminoethanethiol hydrochloride was added thereto, and the mixture was subjected to a reaction at 25°C for 2 hours to introduce 2-aminoethanethiol into sodium alginate. After the reaction, dialysis was performed against water to remove unreacted 2-aminoethanethiol. Finally, a thiol group-introduced sodium alginate solution having a concentration of 1.0% by mass was obtained. The thiol group-introduced sodium alginate is hereinafter referred to as "sodium alginate A1 having a thiol group." An aqueous solution of the sodium alginate A1 having a thiol group is referred to as "thiol group-containing sodium alginate A1 solution." In Examples, both the first binding functional group and the second binding functional group are thiol groups.

(3: Production of Phenol Derivative Solution)

A tyramine hydrochloride phosphate buffer solution having a final concentration of 40 mM was prepared by dissolving tyramine hydrochloride in a phosphate buffer solution (containing 0.01% by mass Tween 20).

(4: Production of Hydrogen Peroxide Aqueous Solution)

A 3.5% by mass hydrogen peroxide aqueous solution was prepared by diluting a hydrogen peroxide aqueous solution (35% by mass) with water.

(5: Production of Specimen Solution containing Peroxidase as Target Substance)

A specimen solution (also referred to as "target substance") having a final concentration of 2 mg/mL (0.05 mM) was prepared by dissolving horseradish peroxidase (HRP) in water.

With the above-mentioned sample production examples, examples of the fluorescence polarization measurement of luminescent particles by a peroxidase enzyme reaction are given in Examples 1 to 6 to be described later.

In a reaction liquid containing a europium complex, the surface of a particle is hydrophilized with Tween 20 and a silane coupling agent (luminescent particle 10). Further, a thiol group (the second binding functional group 20 of the luminescent particle) is added thereto with 2-aminoethanethiol hydrochloride. The sodium alginate (hydrophilic polymer 30 having a first binding functional group) to which the thiol group has been added with 2-aminoethanethiol hydrochloride forms a disulfide crosslinked gel of sodium alginate (aggregate 50 of hydrophilic polymers) by HRP (peroxidase 40), tyramine hydrochloride (phenol derivative) and hydrogen peroxide. When the aggregate of the hydrophilic polymers and the luminescent particle bind to each other via the thiol groups, the mobility of the luminescent particle changes and is detected as a change in degree of fluorescence polarization. Further, in Example 6 to be described later, a measurement example using glucose oxidase (glucose oxidase 60) as a source of hydrogen peroxide is shown.

(Example 1: Fluorescence Polarization Measurement of Sample containing Peroxidase)

Water, the luminescent particle T1 solution, tyramine hydrochloride and the hydrophilic polymer A1 having a thiol group were mixed at the ratios shown in Table 1. A target substance (HRP) was added to the aqueous solution, followed by thorough stirring.

The resultant solution was transferred to a 96-well microplate. Next, a 3.5% by mass hydrogen peroxide aqueous solution was added to the solution in each of the wells, followed by thorough stirring. Then, the microplate was set in a fluorescence polarization measurement apparatus (Nivo multimode microplate reader). A degree of fluorescence polarization (mp) was measured after 5 minutes.

The conditions of the fluorescence polarization measurement apparatus are as follows:

Mode: FP kinetics;
Excitation light: center wavelength of 355 nm/width of 40 nm;
Emission filter: (S) center wavelength of 615 nm/width of 8 nm, (P) center wavelength of 615 nm/width of 8 nm;
Dichroic mirror: D400;
Measurement time: 1,000 ms;
Z-focus: 5 mm;
Measurement spot size: 2 mm on excitation side, 4 mm on emission side;
Flash energy: low (10); and
PMT HV: 1,000.

(Comparative Example 1: Fluorescence Polarization Measurement of Sample free of Peroxidase)

A sample was prepared in the same manner as in Example 1 except that 20 µL of water was added instead of the target substance HRP. A degree of fluorescence polarization (mp) was measured in the same manner as in Example 1.

(Comparative Example 2: Fluorescence Polarization Measurement of Sample free of Hydrogen Peroxide)

A sample was prepared in the same manner as in Example 1 except that the 3.5% by mass hydrogen peroxide aqueous solution was not added. A degree of fluorescence polarization (mp) was measured in the same manner as in Example 1.

(Comparative Example 3: Fluorescence Polarization Measurement of Sample free of Phenol Derivative)

A sample was prepared in the same manner as in Example 1 except that the phenol derivative (tyramine hydrochloride) was not added. A degree of fluorescence polarization (mp) was measured in the same manner as in Example 1.

The results of Example 1 and Comparative Examples 1 to 3 are shown in Table 1.

In the sample free of HRP (Comparative Example 1), the degree of fluorescence polarization was 62.9, but in the sample containing HRP (Example 1), the degree of fluorescence polarization was as large as 97.6. This means that the mobility of the luminescent particle in the sample solution decreased. That is, it is conceived that an aggregate of the hydrophilic polymers was generated in the sample solution, and the binding of the luminescent particle to the aggregate greatly reduced the mobility of the luminescent particle.

When hydrogen peroxide was not added (Comparative Example 2), the degree of fluorescence polarization was as small as 63.8. From this Example, it was found that the addition of hydrogen peroxide was required for rapid measurement of a peroxidase by the fluorescence polarization measurement.

When the phenol derivative (tyramine hydrochloride) was not added (Comparative Example 3), the degree of fluorescence polarization was as small as 63.3. From this Example, it was found that the addition of the phenol derivative (tyramine hydrochloride) was required for rapid measurement of a peroxidase by the fluorescence polarization measurement.

(Table 1)

| | Water | Luminescent particles T1 | Phenol derivative (tyramine hydrochloride) | Hydrogen peroxide aqueous solution | Sodium alginate A1 having thiol group | Target substance HRP | Degree of fluorescence polarization (mp) |
|---|---|---|---|---|---|---|---|
| Stock solution concentration | | 0.08 mg/mL | 40 mM | 3.5% by mass solution | 9 mg/mL | 2 mg/mL | |
| | Reagent composition (μL) | | | | | Target substance (μL) | |
| Example 1 | 100 | 10 | 30 | 5 | 20 | 20 | 97.6 |
| Comparative Example 1 | 100 | 10 | 30 | 5 | 20 | 0 | 62.9 |
| Comparative Example 2 | 100 | 10 | 30 | 0 | 20 | 20 | 63.8 |
| Comparative Example 3 | 100 | 10 | 0 | 5 | 20 | 20 | 63.3 |

(Example 2: Fluorescence Polarization Measurement of Peroxidase)

Water, the luminescent particle T1 solution, tyramine hydrochloride and the sodium alginate A1 having a thiol group were mixed at the ratios shown in Table 2. A target substance (HRP) was added at different concentrations (or activities) to the aqueous solution, followed by thorough mixing. The resultant solutions were each transferred to a 96-well microplate. Next, a 3.5% by mass hydrogen peroxide aqueous solution was added to the solution in each of the wells, followed by thorough mixing. Then, a degree of fluorescence polarization (mp) was measured in the same manner as in Example 1 except that the degree of fluorescence polarization was measured after 10 minutes.

The results of Example 2 are shown in Table 2. As the concentration or activity of HRP increased, the degree of fluorescence polarization increased. It is conceived that HRP generated a sodium alginate aggregate (gel, crosslinked structure), and the binding of the luminescent particle to the sodium alginate aggregate greatly reduced the mobility of the luminescent particle. It was found that by using a relational expression (calibration curve) between the amount (concentration) or activity of the peroxidase and the degree of fluorescence polarization obtained from this Example, the concentration or activity of the peroxidase having an unknown concentration or unknown activity was able to be determined by measuring its degree of fluorescence polarization.

(Table 2)

| Stock solution concentration | | Water | Luminesce nt particles T1 | Phenol derivative (tyramine hydrochloride) | Hydrogen peroxide aqueous solution | Sodium alginate A1 having thiol group | Target substance HRP (20 μL) | | Degree of fluorescence polarization (mp) |
|---|---|---|---|---|---|---|---|---|---|
| | | | 0.08 mg/mL | 40 mM | 3.5% by mass solution | 9 mg/mL | | | |
| | | Reagent composition (μL) | | | | | Final concentration of target substance (μg/mL) | Activity of target substance (U/mL) | |
| Example 2 | | 100 | 10 | 30 | 5 | 20 | 0.0 | 0.00 | 62.6 |
| | | 100 | 10 | 30 | 5 | 20 | 0.1 | 0.02 | 63.1 |
| | | 100 | 10 | 30 | 5 | 20 | 0.9 | 0.18 | 63.5 |
| | | 100 | 10 | 30 | 5 | 20 | 8.0 | 1.60 | 64.8 |
| | | 100 | 10 | 30 | 5 | 20 | 24.0 | 4.80 | 77.4 |
| | | 100 | 10 | 30 | 5 | 20 | 72.1 | 14.41 | 95.8 |
| | | 100 | 10 | 30 | 5 | 20 | 216.2 | 43.24 | 101.4 |

(Example 3: Fluorescence Polarization Measurement of Peroxidase Activity)

Water, the luminescent particle T1 solution, tyramine hydrochloride and the sodium alginate A1 having a thiol group were mixed at the ratios shown in Table 3. A target substance (HRP) having the same concentration, that is, the same activity, was added to the aqueous solution, followed by thorough mixing. A sample obtained by further adding water thereto and another sample obtained by adding a sodium azide aqueous solution, which was an enzyme activity inhibitor, thereto were prepared. Those solutions were each transferred to a 96-well microplate. Next, a 3.5% by mass hydrogen peroxide aqueous solution was added to the solution in each of the wells, followed by thorough mixing. Then, a degree of fluorescence polarization (mp) was measured in the same manner as in Example 1.

The results of Example 3 are shown in Table 3. In the sample in which the sodium azide aqueous solution was added, the degree of fluorescence polarization was small as compared to that of the sample in which the sodium azide aqueous solution was not added. That is, it was found that the decrease in enzyme activity of HRP was able to be measured by the fluorescence polarization measurement.

(Table 3)

| Stock solution concentration | Water | Luminescent particles T1 | Phenol derivative (tyramine hydrochloride) | Hydrogen peroxide aqueous solution | Sodium alginate A1 having thiol group | Target substance HRP (10 $\mu$L) | Enzyme activity inhibitor Sodium azide aqueous solution (0.09% by mass) | Degree of fluorescence polarization (mp) |
|---|---|---|---|---|---|---|---|---|
| | | 0.08 mg/mL | 40 mM | 3.5% by mass solution | 9 mg/mL | | | |
| | Reagent composition ($\mu$L) | | | | | Final concentration of target substance ($\mu$g/mL) | | |
| Example 3 | 100 | 10 | 30 | 1 | 10 | 117.0 | Absent | 100.4 |
| | 100 | 10 | 30 | 1 | 10 | 117.0 | Present | 93.1 |

(Example 4: Fluorescence Polarization Measurement of Hydrogen Peroxide Concentration)

Water, the luminescent particle T1 solution, tyramine hydrochloride, HRP and the sodium alginate A1 having a thiol group were mixed at the ratios shown in Table 4. The resultant solution was transferred to a 96-well microplate. Next, hydrogen peroxide serving as a target substance was added at various concentrations to the solution in each of the wells, followed by thorough stirring. Then, a degree of fluorescence polarization (mp) was measured in the same manner as in Example 1.

The results of Example 4 are shown in Table 4. As the concentration of hydrogen peroxide increased, the degree of fluorescence polarization increased. It is conceived that hydrogen peroxide catalyzed gel formation, and it is presumed that the decrease in mobility of the luminescent particle due to the gel formation occurred in accordance with the amount of hydrogen peroxide.

It was found that by using a relational expression (calibration curve) between the amount (concentration) of hydrogen peroxide and the degree of fluorescence polarization obtained from this Example, the concentration of a hydrogen peroxide aqueous solution having an unknown concentration was able to be determined by measuring its degree of fluorescence polarization.

(Table 4)

| | Water | Luminescent particles T1 | Phenol derivative (tyramine hydrochloride) | HRP | Sodium alginate A1 having thiol group | Target substance Hydrogen peroxide (5 $\mu$L) | Degree of fluorescence polarization (mp) |
|---|---|---|---|---|---|---|---|
| Stock solution concentration | | 0.08 mg/mL | 40 mM | 2 mg/mL | 9 mg/mL | | |
| | Reagent composition ($\mu$L) | | | | | Final concentration of target substance (% by mass) | |
| Example 4 | 100 | 10 | 30 | 20 | 20 | 0.00000 | 64.2 |
| | 100 | 10 | 30 | 20 | 20 | 0.00189 | 67.9 |
| | 100 | 10 | 30 | 20 | 20 | 0.00946 | 89.5 |
| | 100 | 10 | 30 | 20 | 20 | 0.01892 | 99.2 |
| | 100 | 10 | 30 | 20 | 20 | 0.09459 | 101.6 |

(Example 5: Fluorescence Polarization Measurement of Glucose Oxidase)

Water, the luminescent particle T1 solution, tyramine hydrochloride, HRP, the sodium alginate A1 having a thiol group and glucose were mixed at the ratios shown in Table 5. The resultant solution was transferred to a 96-well microplate. Next, glucose oxidase (abbreviated as "GOX") was added as a target substance at various concentrations to the solution in each of the wells, followed by thorough stirring. Then, a degree of fluorescence polarization (mp) was measured in the same manner as in Example 1 except that the degree of fluorescence polarization was measured after 10 minutes.

The results of Example 5 are shown in Table 5. As the concentration of GOX increased, the degree of fluorescence polarization increased.

Hydrogen peroxide is produced from glucose in the solution by the target substance GOX, and the hydrogen peroxide triggers gel formation. It is conceived that the binding of the luminescent particle to the gel increased the degree of fluorescence polarization.

It was found that by using the relational expression (calibration curve) between the GOX concentration and the degree of fluorescence polarization obtained from this Example, the concentration of GOX having an unknown concentration was able to be determined by measuring its degree of fluorescence polarization.

(Table 5)

| | Water | Lumines cent particles T1 | Phenol derivative (tyramine hydrochloride) | HRP | Sodium alginate A1 having thiol group | Glucose | Target substance GOX (50 μL) | Degree of fluorescence polarization (mp) |
|---|---|---|---|---|---|---|---|---|
| Stock solution concentration | | 0.08 mg/mL | 40 mM | 2 mg/mL | 9 mg/mL | 100 mg/mL | | |
| | Reagent composition (μL) | | | | | | Final concentration of target substance (μg/mL) | |
| Example 5 | 100 | 10 | 30 | 20 | 20 | 5 | 0.0 | 64.9 |
| | 100 | 10 | 30 | 20 | 20 | 5 | 7.9 | 70.4 |
| | 100 | 10 | 30 | 20 | 20 | 5 | 118.7 | 75.4 |
| | 100 | 10 | 30 | 20 | 20 | 5 | 237.4 | 79.4 |
| | 100 | 10 | 30 | 20 | 20 | 5 | 395.7 | 82.1 |

(Example 6: Fluorescence Polarization Measurement of Glucose)

A MES buffer solution (pH 5.0), the luminescent particle T1 solution, tyramine hydrochloride, HRP, the sodium alginate A1 having a thiol group and GOX were mixed at the ratios shown in Table 6. The resultant solution was transferred to a 96-well microplate. Next, glucose was added as a target substance at various concentrations to the solution in each of the wells, followed by thorough stirring. Then, a degree of fluorescence polarization (mp) was measured in the same manner as in Example 1 except that the degree of fluorescence polarization was measured after 1 minute.

The results of Example 6 are shown in Table 6. As the concentration of glucose increased, the degree of fluorescence polarization increased. Hydrogen peroxide is generated from the target substance glucose by GOX, and the hydrogen peroxide triggers the gel formation. It is conceived that the binding of the luminescent particle to the gel increased the degree of fluorescence polarization.

It was found that by using the relational expression (calibration curve) between the glucose concentration and the degree of fluorescence polarization obtained from this Example, the concentration of glucose having an unknown concentration was able to be determined by measuring its degree of fluorescence polarization.

(Table 6)

| | MES buffer solution | Lumines cent particles T1 | Phenol derivative (tyramine hydrochloride) | HRP | Sodium alginate A1 having thiol group | GOX | Target substance Glucose (10 µL) | Degree of fluorescence polarization (mp) |
|---|---|---|---|---|---|---|---|---|
| Stock solution concentration | | 0.08 mg/mL | 40 mM | 2 mg/mL | 9 mg/mL | 1.86 mg/mL | | |
| | Reagent composition (µL) | | | | | | Final concentration of target substance (mg/mL) | |
| Example 6 | 100 | 10 | 30 | 20 | 20 | 20 | 0.00 | 63.8 |
| | 100 | 10 | 30 | 20 | 20 | 20 | 0.24 | 67.5 |
| | 100 | 10 | 30 | 20 | 20 | 20 | 0.48 | 69.4 |
| | 100 | 10 | 30 | 20 | 20 | 20 | 2.38 | 71.5 |

Various embodiments have been described in detail above but it will be understood that the present disclosure is not limited to these embodiments and encompasses all modifications, variants, alternatives and equivalents falling within the scope of the appended claims.

**Claims**

1.　A method of detecting a target substance that is a substance related to an enzyme reaction, the method comprising:

a first step of mixing a specimen that may comprise the target substance and a reagent to obtain a liquid sample comprising hydrogen peroxide, a peroxidase (40), a phenol derivative, a luminescent particle (10) and a plurality of hydrophilic polymers (30) each having a binding functional group;
a second step of generating an aggregate (50) of the hydrophilic polymers (30) comprising the luminescent particle (10) through binding of the binding functional groups of the hydrophilic polymers (30) based on a reaction of the hydrogen peroxide, the peroxidase (40) and the phenol derivative, which occurs when the target substance is present in the liquid sample; and
a third step of obtaining a value related to fluorescence anisotropy of the liquid sample in the step of generating the aggregate (50) of the hydrophilic polymers (30) comprising the luminescent particle (10).

2.　The method of detecting a target substance according to claim 1, wherein the binding functional group of each of the hydrophilic polymers is a first binding functional group, and the binding of the binding functional groups is binding of the first binding functional groups to each other.

3.　The method of detecting a target substance according to claim 2, wherein the luminescent particle has a second binding functional group, and the first binding functional group and the second binding functional group bind to each other based on the reaction.

4.　The method of detecting a target substance according to claim 3, wherein the first binding functional group and the second binding functional group are each selected from the group consisting of: a thiol group; a carboxy group; an amino group; and a maleimide group.

5.　The method of detecting a target substance according to claim 4, wherein the first binding functional group and the second binding functional group are each a thiol group.

6.　The method of detecting a target substance according to any one of claims 1 to 5, further comprising a fourth step of detecting the target substance based on the value related to fluorescence anisotropy.

7. The method of detecting a target substance according to any one of claims 1 to 6, wherein the hydrophilic polymers each having the binding functional group are each selected from the group consisting of: alginic acid; hyaluronic acid; gelatin; and polyethylene glycol.

8. The method of detecting a target substance according to claim 3, wherein the aggregate of the hydrophilic polymers comprising the luminescent particle is a conjugate of the aggregate of the hydrophilic polymers and the luminescent particle based on the binding of the first binding functional group and the second binding functional group.

9. The method of detecting a target substance according to any one of claims 1 to 8, wherein the phenol derivative is selected from the group consisting of: phenol; tyramine; tyramine hydrochloride; glycyl-L-tyrosine; resorcinol; and serotonin.

10. The method of detecting a target substance according to any one of claims 1 to 9, wherein the target substance is any one of a peroxidase, hydrogen peroxide and a phenol derivative.

11. The method of detecting a target substance according to any one of claims 1 to 10,

wherein either the target substance is an oxidase and the liquid sample comprises a substrate for the oxidase, or the target substance is a substrate for an oxidase and the liquid sample comprises the oxidase, and wherein the hydrogen peroxide comprises a product generated by the oxidase and the substrate for the oxidase.

12. A reagent for inspection of a target substance using measurement of a value related to fluorescence anisotropy,

wherein the target substance is a peroxidase (40), and the reagent comprises hydrogen peroxide, a phenol derivative, a luminescent particle (10) and a hydrophilic polymer (30) having a binding functional group; wherein the target substance is hydrogen peroxide, and the reagent comprises a peroxidase (40), a phenol derivative, a luminescent particle (10) and a hydrophilic polymer (30) having a binding functional group; or wherein the target substance is a phenol derivative, and the reagent comprises a peroxidase (40), hydrogen peroxide, a luminescent particle (10) and a hydrophilic polymer (30) having a binding functional group.

13. The reagent for inspection of a target substance according to claim 12, wherein the hydrogen peroxide comprises a product generated by the oxidase and the substrate for the oxidase.

14. A reagent for inspection of a target substance using measurement of a value related to fluorescence anisotropy,

wherein the target substance is an oxidase, and the reagent comprises a substrate for the oxidase, a peroxidase (40), hydrogen peroxide, a phenol derivative, a luminescent particle (10) and a hydrophilic polymer (30) having a binding functional group; or wherein the target substance is a substrate for an oxidase, and the reagent comprises the oxidase, a peroxidase (40), hydrogen peroxide, a phenol derivative, a luminescent particle (10) and a hydrophilic polymer (30) having a binding functional group.

# FIG. 1

| |
|---|
| **FIRST STEP**<br>OF MIXING SPECIMEN THAT MAY CONTAIN TARGET SUBSTANCE AND REAGENT TO OBTAIN LIQUID SAMPLE CONTAINING HYDROGEN PEROXIDE, PEROXIDASE, PHENOL DERIVATIVE, LUMINESCENT PARTICLES AND PLURALITY OF HYDROPHILIC POLYMERS EACH HAVING BINDING FUNCTIONAL GROUP |

S1001

↓

| |
|---|
| **SECOND STEP**<br>OF GENERATING AGGREGATE OF HYDROPHILIC POLYMERS INCLUDING LUMINESCENT PARTICLES THROUGH BINDING OF BINDING FUNCTIONAL GROUPS OF HYDROPHILIC POLYMERS BASED ON REACTION OF HYDROGEN PEROXIDE, PEROXIDASE AND PHENOL DERIVATIVE, WHICH OCCURS WHEN TARGET SUBSTANCE IS PRESENT IN LIQUID SAMPLE |

S1002

↓

| |
|---|
| **THIRD STEP**<br>OF OBTAINING VALUE RELATED TO FLUORESCENCE ANISOTROPY OF LIQUID SAMPLE IN STEP OF GENERATING AGGREGATE OF HYDROPHILIC POLYMERS CONTAINING LUMINESCENT PARTICLE |

S1003

↓

| |
|---|
| **FOURTH STEP**<br>OF DETECTING TARGET SUBSTANCE BASED ON VALUE RELATED TO FLUORESCENCE ANISOTROPY |

S1004

EP 4 737 586 A1

# FIG. 2

HIGH MOBILITY

30

20

10

PHENOL DERIVATIVE

HYDROGEN PEROXIDE

（TARGET SUBSTANCE）

40

30

40

10

LOW MOBILITY

50

EP 4 737 586 A1

# FIG. 3

HIGH MOBILITY

40

30

20

10

PHENOL DERIVATIVE

GLUCOSE

60
（TARGET SUBSTANCE）

GENERATION OF HYDROGEN PEROXIDE

60

40

30

50

10

LOW MOBILITY

EP 4 737 586 A1

# FIG. 4

HIGH MOBILITY

HYDROGEN PEROXIDE
(TARGET SUBSTANCE)

PHENOL DERIVATIVE

LOW MOBILITY

FIG. 5

GENERATION OF HYDROGEN PEROXIDE

GLUCOSE
(TARGET
SUBSTANCE)

PHENOL DERIVATIVE

HIGH MOBILITY

LOW MOBILITY

EP 4 737 586 A1

Europäisches
Patentamt

European
Patent Office

Office européen
des brevets

## EUROPEAN SEARCH REPORT

| Application Number |
| --- |
| EP 25 21 2998 |

### DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| A | US 2024/093087 A1 (KAKEGAWA NORISHIGE [JP] ET AL) 21 March 2024 (2024-03-21) * paragraphs [0049]-[0052], [0083], [0085]-[0089], [0092]; the claims * | 1-14 | INV. C12Q1/26 C12Q1/28 G01N33/542 G01N33/58 |
| A | JP H10 99097 A (TOA MEDICAL ELECTRONICS) 21 April 1998 (1998-04-21) * paragraphs [0001], [0014]-[0021]; the claims * | 1-14 | |
| A | US 2024/118207 A1 (KAKEGAWA NORISHIGE [JP] ET AL) 11 April 2024 (2024-04-11) * claims 1-18 * | 1-14 | |
| A | EP 4 276 446 A1 (CANON KK [JP]) 15 November 2023 (2023-11-15) * the whole document, especially the claims; paragraphs [0056], [0073] * | 1-14 | |

| TECHNICAL FIELDS SEARCHED (IPC) |
| --- |
| C12Q G01N |

The present search report has been drawn up for all claims

| Place of search | Date of completion of the search | Examiner |
| --- | --- | --- |
| Munich | 11 March 2026 | Lindberg, Pia |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 21 2998

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2026

| Patent document cited in search report | | Publication date | Patent family member(s) | | Publication date |
|---|---|---|---|---|---|
| US 2024093087 | A1 | 21-03-2024 | CN | 117999308 A | 07-05-2024 |
| | | | EP | 4349887 A1 | 10-04-2024 |
| | | | JP | 2022187791 A | 20-12-2022 |
| | | | US | 2024093087 A1 | 21-03-2024 |
| | | | WO | 2022259989 A1 | 15-12-2022 |
| JP H1099097 | A | 21-04-1998 | NONE | | |
| US 2024118207 | A1 | 11-04-2024 | CN | 117716228 A | 15-03-2024 |
| | | | EP | 4354119 A1 | 17-04-2024 |
| | | | JP | 7728106 B2 | 22-08-2025 |
| | | | JP | 2022187939 A | 20-12-2022 |
| | | | US | 2024118207 A1 | 11-04-2024 |
| | | | WO | 2022259946 A1 | 15-12-2022 |
| EP 4276446 | A1 | 15-11-2023 | EP | 4276446 A1 | 15-11-2023 |
| | | | US | 2023366826 A1 | 16-11-2023 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H10099097 A
- US 20080145880
- US 20170239356
- WO 2015159995 A